# EUROPÄISCHE PATENTSCHRIFT

(11) **EP 2 229 343 B1**
(45) Veröffentlichungstag und Bekanntmachung des Hinweises auf die Patenterteilung: **21.09.2011**
(21) Anmeldenummer: 08856513.0
(22) Anmeldetag: 22.11.2008
(51) Int. Cl.: C01D 3/06, C07C 68/06, C25B 1/34

(54) **VERFAHREN ZUR HERSTELLUNG VON DIARYLCARBONAT**
PROCESS FOR THE PREPARATION OF DIARYL CARBONATE
PROCEDE DE FABRICATION DE DIARYLCARBONATE

(30) Priorität: 06.12.2007 DE 102007058701
(43) Veröffentlichungstag der Anmeldung: 22.09.2010
(73) Patentinhaber: Bayer MaterialScience AG, 51368 Leverkusen (DE)
(72) Erfinder: OOMS, Pieter, 47800 Krefeld (DE); BULAN, Andreas, 40764 Langenfeld (DE); RECHNER, Johann, 47906 Kempen (DE); WEBER, Rainer, 51519 Odenthal (DE); BUTS, Marc, B-2570 Duffel (BE); VANDEN EYNDE, Johan, 9052 Zwijnaarde (BE)
(86) Internationale Anmeldenummer: PCT/EP2008/009910
(87) Internationale Veröffentlichungsnummer: WO 2009/071211

(56) Entgegenhaltungen:
- EP-A- 1 216 982
- EP-A- 1 894 914
- WO-A-00/78682
- WO-A-01/38419
- WO-A-03/070639
- DE-A1-102004 041 777

## Beschreibung

Die Erfindung betrifft ein Verfahren zur Herstellung von Diarylcarbonat kombiniert mit der Elektrolyse des erhaltenen Alkalichlorid-haltigen Prozessabwassers. Das erfindungsgemäße Verfahren ermöglicht unter anderem eine verbesserte Verwertung der aus der Herstellung des Diarylcarbonats erhaltenen Alkalichlorid-haltigen Lösung in der Elektrolyse.

Die Herstellung von Diarylcarbonaten (Diarylcarbonat) erfolgt üblicherweise durch ein kontinuierliches Verfahren, durch Herstellung von Phosgen und anschließende Reaktion von Monophenolen und Phosgen in einem inerten Lösungsmittel in Gegenwart von Alkali und einem basischen Stickstoffkatalysator in der Grenzfläche.

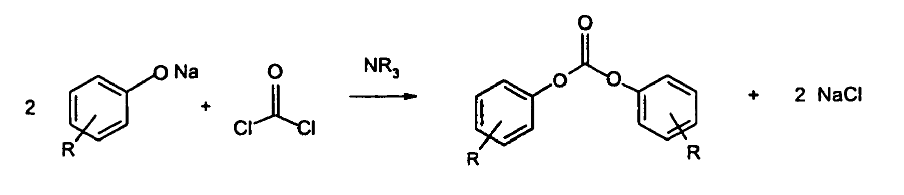

Die Herstellung von Diarylcarbonaten z.B. durch den Phasengrenzflächenprozess ist prinzipiell in der Literatur beschrieben, siehe z.B. in Chemistry and Physics of Polycarbonates, Polymer Reviews, H. Schnell, Vol. 9, John Wiley and Sons, Inc. (1964), S. 50/51.

In der US-A-4 016 190 wird ein Herstellungsverfahren von Diarylcarbonaten beschrieben, das bei Temperaturen >65°C betrieben wird. Der pH-Wert wird bei diesem Verfahren zunächst niedrig (pH 8 bis 9) und anschließend hoch (10 bis 11) eingestellt.

Optimierungen des Verfahrens durch Verbesserung der Durchmischung und Einhaltung eines engen Temperatur- und pH-Profils als auch Isolierung des Produkts werden in EP 1 219 589 A1, EP 1 216 981 A2, EP 1 216 982 A2 und EP 784 048 A1 beschrieben.

Bei diesen bekannten Verfahren macht jedoch ein hoher Restphenolwert im Abwasser dieser Prozesse, welche die Umwelt belasten können und die Klärwerke vor eine erhöhte Abwasserproblematik stellen, aufwendige Reinigungsoperationen erforderlich. So beschreibt WO 03/070639 A1 eine Entfernung der organischen Verunreinigungen im Abwasser durch eine Extraktion mit Methylenchlorid.

Üblicherweise wird die Alkalichlorid-haltige, vorzugsweise Natriumchlorid-haltige Lösung von Lösungsmitteln und organischen Resten befreit und muss dann entsorgt werden.

Bekannt ist aber auch, dass die Reinigung der Natriumchlorid-haltigen Abwässer gemäß der EP 1 200 359 A 1 oder US-A-6 340 736 durch Ozonolyse erfolgen kann und dann zum Einsatz bei der Natriumchlorid-Elektrolyse geeignet ist. Nachteil der Ozonolyse ist, dass dieses Verfahren sehr kostenintensiv ist.

Gemäß der EP 541 114 A2 wird ein Natriumchlorid-haltiger Abwasserstrom bis zur vollständigen Entfernung des Wassers eingedampft und das zurückbleibende Salz mit den organischen Verunreinigungen einer thermischen Behandlung unterzogen, wodurch die organischen Bestandteile zersetzt werden. Besonders bevorzugt ist dabei der Einsatz von Infrarotstrahlung. Nachteil des Verfahrens ist, dass das Wasser vollständig verdampft werden muss, so dass das Verfahren wirtschaftlich nicht durchführbar ist.

Gemäß der WO 03/70639 A1 wird das Abwasser aus einer DPC-Produktion durch Extraktion gereinigt und dann der Natriumchlorid-Elektrolyse zugeführt. Durch das beschriebene Verfahren können jedoch nur maximal 26 % des Natriumchlorids aus dem Abwasser der DPC-Produktion zurück gewonnen werden, da bei größeren Einsatzmengen das in die Elektrolyse mit dem Abwasser eingebrachte Wasser die Wasserbilanz der Natriumchlorid-Elektrolyse aus dem Gleichgewicht bringen würde.

Die Alkalichlorid-haltigen, vorzugsweise Natriumchlorid-haltigen Lösungen, die bei der DPC-Produktion anfallen, haben typischerweise einen Alkalichlorid-Gehalt, vorzugsweise Natriumchlorid-Gehalt von 13 bis 17 Gew.%. Somit ist es nicht möglich, das in den Lösungen vorhandene gesamte Alkalichlorid auf diesem Wege wieder zu gewinnen. Bei einer Alkalichlorid-Konzentration von 17 Gew.% gelingt bei der Standard-Alkalichlorid-Elektrolyse, vorzugsweise Standard-Natriumchlorid-Elektrolyse mit einer handelsüblichen Ionenaustauschermembran, die einen Wassertransport von 3,5 mol Wasser je mol Natrium aufzeigt, nur der Einsatz von ca. 23 % des Natriumchlorids aus dem Natriumchlorid-haltigen Lösungen. Selbst bei Aufkonzentrierung bis zu einer gesättigten Natriumchlorid-Lösung von ca. 25 Gew.% gelänge nur eine Recyclingquote von 38 % des in der Natriumchlorid-haltigen Lösung enthaltenden Natriumchlorids. Ein vollständiges Recycling der Alkalichlorid-haltigen Lösung ist bisher nicht bekannt. Gemäß der WO 01/38419 A1 kann die Natriumchlorid-haltige Lösung mittels thermischer Verfahren eingedampft werden, so dass eine hoch konzentrierte Natriumchlorid-Lösung der Elektrolysezelle zugeführt werden kann. Die Eindampfung ist jedoch energieintensiv und kostspielig.

Ausgehend vom oben geschilderten Stand der Technik bestand die Aufgabe der Erfindung darin, ein Verfahren zur Herstellung von Diarylcarbonat bereitzustellen, welches Produkte in hoher Reinheit und guter Ausbeute liefert und gleichzeitig eine Reduzierung der Umweltbelastung bzw. Abwasserproblematik in den Klärwerken durch maximierte Rückführung von Alkalichlorid aus Alkalichlorid-haltigen Prozessabwasserlösungen, die aus der Diarylcarbonat-Produktion erhalten werden, ermöglicht.

Insbesondere sollte bei dem Recycling berücksichtigt werden, dass die Umsetzung von Alkalichlorid, vorzugsweise Natriumchlorid zu Chlor und Alkalilauge, vorzugsweise Natronlauge und gegebenenfalls Wasserstoff mit minimalem Energieeinsatz und daher ebenfalls Resourcenschonend erfolgen sollte.

Es wurde überraschend gefunden, dass bei der Herstellung von Diarylcarbonaten durch Reaktion von Monophenolen und Phosgen in einem inerten Lösungsmittel in Gegenwart einer Base und gegebenenfalls einem basischen Katalysator eine verbesserte Verwertung der aus der Herstellung des Diarylcarbonats erhaltenen Alkalichlorid-haltigen Lösung in einer Chlor-Alkali-Elektrolyse erreicht werden kann, wenn die bei der Herstellung von Diarylcarbonaten erhaltene Alkalichlorid-haltige Lösung einen Alkalichlorid-Gehalt von 18 bis 25 Gew.% bezogen auf das Gesamtgewicht der Alkalichlorid-haltigen Lösung aufweist. Einen solche Alkalichlorid-Gehalt von 18 bis 25 Gew.-% der bei der Herstellung von Diarylcarbonaten erhaltenen Alkalichlorid-haltigen Lösung kann erfindungsgemäß sowohl dadurch erzielt werden, dass bei der Umsetzung von Monophenol und Phosgen in Gegenwart einer Base das Monophenol und eine alkalihaltige Base in solchen Mengen eingesetzt werden, dass der Natriumphenolatgehalt der resultierenden Lösung aus Alkali-haltiger Base und Monophenol in einem speziell ausgewählten Bereich liegt, bevorzugt von 31 bis 40 Gew.% Natriumphenolat, bezogen auf das Gesamtgewicht der Lösung. Zusätzlich oder alternativ kann wenigstens ein Teil der bei der Herstellung von Diarylcarbonaten erhaltene Alkalichlorid-haltige Lösung, in die Herstellung von Diarylcarbonaten zurückgeführt werden, beispielsweise durch Ersatz etwaig zu verwendenden Wassers, um ebenfalls eine Aufkonzentrierung der bei der Herstellung von Diarylcarbonaten erhaltenen Alkalichlorid-haltigen Lösung auf einen Alkalichlorid-Gehalt von 18 bis 25 Gew.-% zu erreichen.

Dies ist überraschend, da eine Erhöhung des Natriumphenolatgehalts in der Ausgangslösung bei der Herstellung von Diarylcarbonaten bei der nachfolgenden exothermen Bildung von Diarylcarbonaten zu erhöhtem Energieaufkommen und folglich zu verstärkter Nebenproduktbildung führen sollte. Zudem musste bei der Rückführung der Natriumchlorid-haltigen Lösung in die Diarylcarbonat-Herstellung, beispielsweise durch Ersatz etwaig zu verwendenden Wassers, durch die Anwesenheit von Alkalichlorid in der Eduktlösung mit einer Verringerung der Löslichkeit des Phenols und entsprechender Ausfällung gerechnet werden. Beides wurde innerhalb des erfindungsgemäß ausgewählten Bereiches eines Alkalichlorid-Gehalt von 18 bis 25 Gew.-% bezogen auf das Gesamtgewicht der Alkalichlorid-haltigen Lösung überraschend nicht beobachtet. Hingegen bietet das erfindungsgemäße Verfahren überraschend die Möglichkeit, deutlich größere Anteile des im Reaktionsabwasser der Diarylcarbonatherstellung befindlichen Alkalichlorids mittels Elektrolyse zu rezyclieren und damit wieder zu verwerten. Gegenstand der Erfindung ist ein Verfahren zur Herstellung von Diarylcarbonat und Verarbeitung mindestens eines Teils der dabei anfallenden Alkalichlorid-haltigen Lösung in einer nachgeschalteten Alkalichlorid-Elektrolyse, umfassend die folgenden Schritte:
a) Herstellung von Phosgen durch Umsetzung von Chlor mit Kohlenmonoxid, und
b) Umsetzung des gemäß Schritt a) gebildeten Phosgens mit wenigstens einem Monophenol in Gegenwart einer Base und gegebenenfalls basischem Katalysator zu einem Diarylcarbonat und einer Alkalichlorid-haltigen Lösung, und
c) Abtrennung und Aufarbeitung des in Schritt b) gebildeten Diarylcarbonats, und
d) Abtrennung der gemäß Schritt c) verbliebenen Alkalichlorid-haltigen Lösung von Lösungsmittelresten und gegebenenfalls Katalysatorresten, insbesondere durch Strippen der Lösung mit Wasserdampf, und Behandlung mit Adsorbentien, insbesondere mit Aktivkohle, wobei vor der Behandlung mit Adsorbentien die Alkalichlorid-haltige Lösung auf einen pH-Wert kleiner oder gleich 8 eingestellt wird, und
e) Elektrochemische Oxidation wenigstens eines Teils der Alkalichlorid-haltigen Lösung aus d) unter Bildung von Chlor, Alkalilauge und ggf. Wasserstoff, und
f) Rückführung wenigstens eines Teil des gemäß Schritt e) hergestellten Chlors in die Herstellung von Phosgen gemäß Schritt a) und/oder
g) Rückführung wenigstens ein Teil des gemäß Schritt e) hergestellten Alkalilauge in die Herstellung von Diarylcarbonat gemäß Schritt b),
dadurch gekennzeichnet, dass die in Schritt b) hergestellte Alkalichlorid-haltige Lösung einen Alkalichlorid-Gehalt von 18 bis 25 Gew.% bezogen auf das Gesamtgewicht der Alkalichlorid-haltigen Lösung hat und/oder wenigstens ein Teil der in Schritt d) anfallenden Alkalichlorid-haltigen Lösung in Schritt b) zurückgeführt wird.

Der im erfindungsgemäßen Verfahren für die Elektrolyse wünschenswerte erhöhte Alkalichlorid-Gehalt von 18 bis 25 Gew.-% bezogen auf das Gesamtgewicht der Alkalichlorid-haltigen Lösung kann dadurch erreicht werden, dass in Schritt b) als Base eine Alkali-haltige Base, bevorzugt eine Natrium-haltige Base, eingesetzt wird und diese Alkali-haltige Base und Monophenol in solchen Mengen eingesetzt werden, dass der Natriumphenolatgehalt der resultierenden Lösung aus Alkali-haltiger Base und Monophenol 31 bis 40 Gew.-% Natriumphenolat, bezogen auf das Gesamtgewicht der Lösung, beträgt und/oder wenigstens ein Teil der in Schritt d) anfallenden Alkalichlorid-haltigen Lösung in Schritt b) zurückgeführt wird. Dabei können bevorzugt bis zu 80 Gew.%, bevorzugt bis zu 50 Gew.-% der in Schritt d) anfallenden Alkalichlorid-haltigen Lösung in Schritt b) zurückgeführt werden.

Für den Fall, dass wenigstens ein Teil der in Schritt d) anfallenden Alkalichlorid-haltigen Lösung in Schritt b) zurückgeführt wird, kann in bevorzugten Ausführungsformen des erfindungsgemäßen Verfahrens die gemäß Schritt c) verbliebene Alkalichlorid-haltige Lösung zudem wenigstens teilweise mit Waschwasser aus der Aufarbeitung des Diarylcarbonats gemäß Schritt c) vereinigt in Schritt d) eingesetzt werden. Diese Vorgehensweise bietet den zusätzlichen Vorteil, dass die vollständige Entsorgung der Waschphasen vermieden werden kann.

Vorzugsweise wird in Schritt b) der Natriumphenolatgehalt der resultierenden Lösung aus Alkali-haltiger Base und Monophenol von 31 bis 40 Gew.-% Natriumphenolat durch Umsetzung von einem Monophenol mit einer 14 bis 21 Gew.%-igen Alkali-haltigen Base, insbesondere einer Natrium-haltigen Base, erzielt.

Besonders geeignete Monophenole zum Einsatz in dem neuen Verfahren sind Phenole der Formel (I) 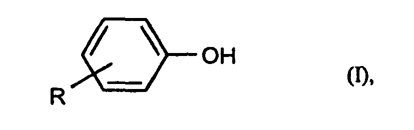 worin
- R: Wasserstoff, Halogen oder ein verzweigter oder unverzweigter C₁- bis C₉-Alkylrest oder Alkoxycarbonylrest ist.

C₁-C₉-Alkyl steht im Rahmen der Erfindung beispielsweise und bevorzugt für Methyl, Ethyl, n-Propyl, iso-Propyl, n-Butyl, sec.-Butyl, tert.-Butyl, n-Pentyl, 1-Methylbutyl, 2-Methylbutyl, 3-Methylbutyl, neo-Pentyl, 1-Ethylpropyl, cyclo-Hexyl, cyclo-Pentyl, n-Hexyl, n-Heptyl, n-Octyl, iso-Octyl, n-Nonyl, iso-Nonyl.

Halogen steht im Rahmen der Erfindung beispielsweise und bevorzugt für Fluor, Chlor, Brom oder Iod, bevorzugt für Chlor oder Brom.

Bevorzugte geeignete Monophenole sind also Phenol, Alkylphenole wie Kresole, p-tert.-Butylphenol, p-Cumylphenol, p-n-Octylphenol, p-iso-Octylphenol, p-n-Nonylphenol und p-isoNonylphenol, Halogenphenole wie p-Chlorphenol, 2,4-Dichlorphenol, p-Bromphenol und 2,4,6-Tribromphenol oder Salicylsäuremethylester. Besonders bevorzugt ist Phenol.

Als Base für die Umsetzung des Monophenols mit Phosgen eignen sich beispielsweise Alkalisalze, bevorzugt Alkalihydroxide, wie z.B. Na-, K- oder Li-Hydroxid, besonders bevorzugt Natronlauge. Die Base wird im erfindungsgemäßen Verfahren vorzugsweise als 14 bis 21 Gew.%-ige wässrige Lösung eingesetzt.

Die Umsetzung in Schritt b) kann durch basische Katalysatoren wie tertiäre Amine, N-Alkylpiperidine oder Oniumsalze beschleunigt werden. Bevorzugt sind stickstoff-haltige Katalysatoren. Besonders bevorzugt werden Tributylamin, Triethylamin und N-Ethylpiperidin verwendet.

Der eingesetzte basische Katalysator kann offenkettig oder cyclisch sein, besonders bevorzugt ist Triethylamin und Ethylpiperidin. Der Katalysator wird im erfindungsgemäßen Verfahren vorzugsweise als 1 bis 55 Gew.%-ige Lösung eingesetzt.

Unter Oniumsalzen werden im Rahmen der Erfindung vorzugsweise Verbindungen wie NR₄X verstanden, wobei die Reste R unabhängig voneinander für H und/oder ein Alkyl und/oder Arylrest stehen können und X ein Anion, wie z.B. Chlorid, Bromid oder Iodid, ist.

Phosgen kann in Schritt b) flüssig, gasförmig oder gelöst in einem inerten Lösungsmittel eingesetzt werden.

Im erfindungsgemäßen Verfahren in Schritt b) bevorzugt verwendbare inerte organische Lösungsmittel sind vorzugsweise aromatische Lösungsmittel, halogenierte, bevorzugt chlorierte aliphatische oder aromatische Lösungsmittel oder Mischungen aus diesen. Dies sind beispielsweise Toluol, Dichlormethan, die verschiedenen Dichlorethane und Chlorpropanverbindungen, Chlorbenzol und Chlortoluol oder Mischungen aus diesen. Besonders bevorzugt wird Dichlormethan eingesetzt.

Im erfindungsgemäßen Verfahren in Schritt b) hergestellte Diarylcarbonate sind bevorzugt solche der allgemeinen Formel (II) 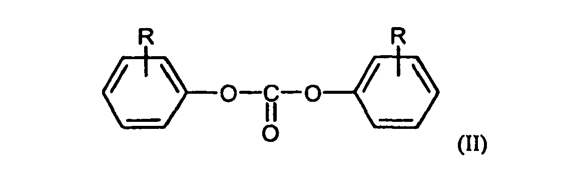 worin R die für die Formel (I) genannte Bedeutung hat. Besonders bevorzugt wird mit dem erfindungsgemäßen Verfahren Diphenylcarbonat hergestellt.

Die Reaktionsführung für Schritt b) erfolgt bevorzugt kontinuierlich und besonders bevorzugt in einer Pfropfenströmung ohne große Rückvermischung. Dies kann beispielsweise in Rohrreaktoren geschehen. Die Durchmischung der zwei Phasen (wässrige und organische Phase) kann beispielsweise durch eingebaute Rohrblenden, statische Mischer und/oder Pumpen realisiert werden.

Die Reaktion gemäß Schritt b) erfolgt besonders bevorzugt zweistufig.

In der ersten Stufe des bevorzugten Verfahrens wird die Reaktion durch Zusammenbringen der Edukte Phosgen, wenigstens eines inerten organischen Lösungsmittels, welches bevorzugt erst als Lösungsmittel für das Phosgen dient, und des Monophenols, welches vorzugsweise zuvor bereits in der Lösung der Base, vorzugsweise in der Alkalilauge gelöst ist, gestartet. Die Verweilzeit liegt typischerweise in der ersten Stufe im Bereich von 2 Sekunden bis 300 Sekunden, besonders bevorzugt im Bereich von 4 Sekunden bis 200 Sekunden. Der pH-Wert der ersten Stufe wird durch das Verhältnis von Base (bevorzugt Alkalilauge) /Monophenol /Phosgen bevorzugt so eingestellt, dass der pH-Wert im Bereich von 11,0 bis 12,0, bevorzugt 11,2 bis 11,8, besonders bevorzugt bei 11,4 bis 11,6 liegt. Die Reaktionstemperatur der ersten Stufe wird durch Kühlung bevorzugt unter 40°C, besonders bevorzugt auf 35°C oder darunter gehalten.

In der zweiten Stufe des bevorzugten Verfahrens wird die Reaktion zum Diarylcarbonat komplettiert. Die Verweilzeit beträgt beim bevorzugten Prozess 1 Minute bis 2 Stunden, bevorzugt 2 Minuten bis 1 Stunde, ganz besonders bevorzugt 3 Minuten bis 30 Minuten. In der zweiten Stufe des bevorzugten Verfahrens wird durch permanente Kontrolle des pH-Werts, welcher im kontinuierlichen Verfahren bevorzugt Online nach grundsätzlich bekannten Verfahren gemessen wird, und entsprechende Einstellung des pH-Wertes durch Zugabe der Alkalilauge geregelt. Die Menge der zugeführten Base, vorzugsweise Alkalilauge, wird insbesondere so eingestellt, dass der pH-Wert der Reaktionsmischung in der zweiten Verfahrensstufe im Bereich von 7,5 bis 10,5, bevorzugt 8 bis 9,5, ganz besonders bevorzugt 8,2 bis 9,3 liegt. Die Reaktionstemperatur der zweiten Stufe wird durch Kühlung bei bevorzugt unter 50°C, besonders bevorzugt unter 40°C, ganz besonders bevorzugt 35°C oder darunter gehalten. Die in Schritt b) hergestellte Alkalichlorid-haltige Lösung weist demnach unmittelbar nach Komplettierung der Reaktion zum Diarylcarbonat vorzugsweise eine Temperatur von unter 50°C, besonders bevorzugt unter 40°C, ganz besonders bevorzugt 35°C oder darunter auf.

Die in dieser Anmeldung aufgeführten allgemeinen oder in Vorzugsbereichen aufgeführten Parameter bzw. Erläuterungen können auch untereinander, also zwischen den jeweiligen Bereichen und Vorzugsbereichen beliebig kombiniert werden.

Im bevorzugten Ausführungsformen des erfindungsgemäßen Verfahrens wird in Schritt b) Phosgen in Bezug auf das Monophenol im Mol-Verhältnis von 1 : 2 bis 1 : 2,2 eingesetzt. Das inerte organische Lösungsmittel wird so zugemischt, dass das Diarylcarbonat nach der Reaktion in einer 5 bis 50 Gew.-%igen Lösung, bevorzugt 20 bis 45 Gew.%igen Lösung, bezogen auf die organische Phase, vorliegt.

Der gegebenenfalls eingesetzte Katalysator wird bevorzugt in Mengen von 0,0001 mol bis 0,1 mol, bezogen auf das eingesetzte Monophenol, zugegeben.

Nach der Reaktion b) wird in Schritt c) bevorzugt die organische, das Diarylcarbonat enthaltende Phase üblicherweise mit einer wässrigen Flüssigkeit gewaschen und nach jedem Waschvorgang von der wässrigen Phase soweit wie möglich getrennt. Als Waschflüssigkeit werden wässrige Flüssigkeiten zur Abtrennung des Katalysators, z.B. verdünnte Mineralsäuren wie HCl oder H₃PO₄, und zur weiteren Reinigung vollentsalztes Wasser eingesetzt. Die Konzentration von HCl oder H₃PO₄ in der Waschflüssigkeit kann vorzugsweise 0,5 bis 1,0 Gew.-% betragen. Die organische Phase wird beispielhaft und vorzugsweise zweimal gewaschen.

Als Phasentrennvorrichtungen zur Abtrennung der Waschflüssigkeit von der organischen Phase können grundsätzlich bekannte Trenngefäße, Phasenseparatoren, Zentrifugen oder Coalescer oder auch Kombinationen dieser Einrichtungen verwendet werden.

Man erhält so ohne Berücksichtigung des noch abzutrennenden Lösungsmittels überraschend hohe Reinheitsgrade des Diarylcarbonats von >99,85 %.

Nach der Synthese des Diarylcarbonats wird das Diarylcarbonat in Form seiner Lösung in dem bei der Herstellung verwendeten inerten organischen Lösungsmittel, beispielsweise Methylenchlorid, abgetrennt.

Zum Erhalt des hochreinen Diarylcarbonats wird das Lösungsmittel vorzugsweise verdampft. Das Verdampfen kann in mehreren Verdampferstufen erfolgen. Beispielsweise erfolgt dies durch eine oder mehrere hintereinander geschaltete Destillationskolonnen bei der das Lösungsmittel vom Diarylcarbonat abgetrennt wird.

Diese Reinigungsstufe(n) c) können beispielsweise kontinuierlich so geführt werden, dass die Sumpftemperatur bei der Destillation 150°C bis 310°C, bevorzugt 160 bis 230°C beträgt. Der zur Durchführung dieser Destillation angewendete Druck beträgt dabei insbesondere 1 bis 1000 mbar, bevorzugt 5 bis 100 mbar.

Die so gereinigten Diarylcarbonate zeichnen sich durch besonders hohe Reinheit (gemäß GC-Analyse >99,95%) und extrem gutes Umesterungsverhalten aus, so dass diese sich zur anschließend Herstellung von Polycarbonaten exzellenter Qualität eignen. Die Verwendung der Diarylcarbonate zur Herstellung von aromatischen Oligo-/Polycarbonaten nach dem Schmelzumesterungsverfahren ist literaturbekannt und beispielsweise in der Encyclopedia of Polymer Science, Vol. 10 (1969), Chemistry and Physics of Polycarbonates, Polymer Reviews, H. Schnell, Vol. 9, John Wiley and Sons, Inc. (1964) oder der US-A- 5 340 905 beschrieben.

Die nach der Phasentrennung im Rahmen der Abtrennung gemäß Schritt c) verbleibende wässrige Alkalichlorid-haltige Lösung - im Folgenden auch als Alkalichlorid-haltiges Abwasser bezeichnet - wird vorteilhafterweise von leicht flüchtigen organischen Verunreinigungen, wie z.B. Resten des bei der Synthese verwendeten organischen Lösungsmittels und gegebenenfalls Katalysatorresten beispielsweise durch Destillation oder Wasserdampfstrippen befreit. Dabei verbleibt eine Alkalichlorid-haltige Lösung mit einem hohen Gehalt an gelöstem Alkalichlorid von 18 bis 25 Gew.-% bezogen auf das Gesamtgewicht der Alkalichlorid-haltigen Lösung, welche zudem noch gelöste Alkalicarbonate in einer Menge von 0,3 bis 1,5 Gew.-% bezogen auf das Gesamtgewicht der Alkalichlorid-haltigen Lösung enthält. Diese Alkalicarbonate können z.B. durch Hydrolyse des Phosgens als Nebenreaktion der Diarylcarbonatherstellung entstanden sein. Außerdem ist das Alkalichlorid-haltige Abwasser mit organischen Verbindungen belastet, z.B. mit Phenolen (z.B. unsubstituiertes Phenol, Alkylphenole).

Anschließend erfolgt die Behandlung des vorgereinigten Alkalichlorid-haltigen Abwassers mit Adsorbentien, bevorzugt mit Aktivkohle. Vor dieser Behandlung mit Adsorbentien wird das Alkalichlorid-haltige Abwasser auf einen pH-Wert kleiner oder gleich 8, bevorzugt auf einen pH-Wert von kleiner als 7 eingestellt.

Diese Erniedrigung des pH-Wertes im Prozessschritt d) erfolgt vorzugsweise durch Zugabe von Protonensäuren. Bevorzugt wird die Erniedrigung des pH-Wertes im Prozessschritt d) mit Salzsäure oder gasförmigem Chlorwasserstoff durchgeführt. Der grundsätzlich denkbare, aber im vorliegenden Verfahren unerwünschte Einsatz der preiswerteren Schwefelsäure würde dazu führen, dass bei der pH-Erniedrigung Natriumsulfat entstünde, welches bei der anschließenden Elektrolyse im Anolytkreislauf angereichert würde. Da z.B. die Ionenaustauschermembranen gemäß Herstellerangaben nur bis zu einer bestimmten Natriumsulfat-Konzentration im Anolyten betrieben werden dürfen, müsste mehr Anolyt ausgeschleust werden als bei Einsatz von Salzsäure oder Chlorwasserstoff, dessen Reaktionsprodukt zudem vorteilhafterweise wiederum das gewünschte Natriumchlorid ist.

Wenigstens wenigstens ein Teils des Alkalichlorid-haltigen Abwassers aus Schritt d) wird anschließend einer elektrochemische Oxidation, vorzugsweise in Form einer Alkaliclorid-Elektrolyse unter Bildung von Chlor, Alkalilauge und gegebenenfalls Wasserstoff unterzogen.

Das Verfahren der Alkalichlorid-Elektrolyse wird im Folgenden näher beschrieben. Die nachstehende Beschreibung ist in Bezug auf die Elektrolyse von Natriumchlorid beispielhaft anzusehen, da im Verfahren wie bereits oben ausgerührt wurde grundsätzlich jedes Alkalichlorid zum Einsatz kommen kann (insbesondere Li-, Na-, KCl). Da die Verwendung von Natriumchlorid bzw. Natronlauge in den vorgehenden Stufen des erfindungsgemäßen Verfahrens jedoch bevorzugt ist, ist auch die Elektrolyse von Natriumchlorid die bevorzugte Ausführungsform des erfindungsgemäßen Verfahrens.

Üblicherweise werden z.B. zur Elektrolyse von Natriumchlorid-haltigen Lösungen Membranelektrolyseverfahren eingesetzt (siehe hierzu Peter Schmittinger, CHLORINE, Wiley-VCH Verlag, 2000). Hierbei wird eine zweigeteilte Elektrolysezelle eingesetzt, die aus einem Anodenraum mit einer Anode und einem Kathodenraum mit einer Kathode besteht. Anoden- und Kathodenraum werden von einer Ionenaustauschermembran getrennt. In den Anodenraum wird eine Natriumchlorid-haltige Lösung mit einer Natriumchlorid-Konzentration von üblicherweise mehr als 300 g/l eingeführt. An der Anode wird das Chlorid-Ion zu Chlor oxidiert, welches mit der abgereicherten Natriumchlorid-haltigen Lösung aus der Zelle geführt wird. Die Natrium-Ionen wandern unter Einfluss des elektrischen Feldes durch die Ionenaustauschermembran in den Kathodenraum. Bei dieser Wanderung nimmt jedes mol Natrium je nach Membran zwischen 3,5 und 4,5 mol Wasser mit. Dies führt dazu, dass der Anolyt an Wasser verarmt. Im Gegensatz zum Anolyten wird auf der Kathodenseite durch die Elektrolyse von Wasser zu Hydroxid-Ionen und Wasserstoff Wasser verbraucht. Das mit den Natrium-Ionen in den Katholyten gelangende Wasser reicht aus, um die Natronlauge-Konzentration im Auslauf auf 31 bis 32 Gew.-% - bei einer Einlaufkonzentration von 30% und einer Stromdichte von 4 kA/m² - zu halten. Im Kathodenraum wird Wasser elektrochemisch reduziert, wobei Hydroxid-Ionen und Wasserstoff entstehen.

Alternativ kann als Kathode auch eine Gasdiffusionselektrode eingesetzt werden, an der Sauerstoff mit Elektronen zu Hydroxid-Ionen umgesetzt wird, wobei kein Wasserstoff entsteht. Mit den über die Ionenaustauschermembran in den Kathodenraum gelangten Natrium-Ionen bilden die Hydroxid-Ionen Natronlauge. In die Kathodenkammer wird üblicherweise eine Natronlauge mit einer Konzentration von 30 Gew.-% zugeführt und eine Natronlauge mit einer Konzentration von 31 bis 32 Gew.-% abgeführt. Ziel ist es eine möglichst hohe Konzentration an Natronlauge zu erreichen, da üblicherweise die Natronlauge als 50 Gew.-%ige Lauge gelagert oder transportiert wird. Handelsübliche Membranen sind jedoch derzeit nicht beständig gegen eine Lauge mit einer höheren Konzentration als 32 Gew.%, so dass die hergestellte Natronlauge nachträglich durch thermische Eindampfung aufkonzentriert werden muss.

Im Falle der Natriumchlorid-Elektrolyse im Rahmen des erfindungsgemäßen Verfahrens wird über den Einsatz des Natriumchlorid-haltigen Abwassers zusätzliches Wasser in den Anolyten eingebracht, jedoch nur Wasser über die Membran in den Katholyten ausgetragen. Wird mehr Wasser über die Natriumchlorid-haltige Lösung eingebracht als zum Katholyten transportiert werden kann, verarmt der Anolyt an Natriumchlorid und die Elektrolyse kann nicht kontinuierlich betrieben werden. Bei sehr geringen Natriumchlorid-Konzentrationen würde die Nebenreaktion der Sauerstoffbildung einsetzen.

Um maximale Mengen an Natriumchlorid-haltigen Lösungen wirtschaftlich der Natriumchlorid-Elektrolyse zuzuführen, kann es nützlich sein, dass der Wassertransport über die Membran erhöht wird. Dieser kann durch Auswahl geeigneter Membranen erfolgen, wie in der US-A-4025405 beschrieben. Der Effekt eines erhöhten Wassertransportes ist, dass auf die sonst übliche Wasserzugabe zur Aufrechterhaltung der Laugekonzentration verzichtet werden kann.

Gemäß der US-A-3 773 634 kann bei hohem Wassertransport durch die Membran die Elektrolyse dann betrieben werden, wenn eine Laugekonzentration von 31 bis 43 Gew.-% und eine Natriumchlorid-Konzentration von 120 bis 250 g/l eingesetzt wird.

Nachteil beider Verfahren ist die niedrigere Stromausbeute dieser Verfahren.

Gemäß dem erfindungsgemäßen Verfahren erfolgt die Abtrennung c) des Natriumchlorid-haltigen Abwassers mittels Phasentrennung. Anschließend erfolgt im Schritt d) Entfernung des Lösungsmittels und gegebenenfalls verwendeten Katalysators, insbesondere durch Strippung mit Wasserdampf, sowie nach pH-Einstellung durch eine Behandlung mit Adsorbentien. Hiernach kann das Alkalichlorid-haltige Abwasser direkt der Elektrolyse e) zugeführt werden.

In einer bevorzugten Ausführungsform des erfindungsgemäßen Verfahrens kann dem Alkalichlorid-haltigen Abwasser durch ein Aufkonzentrierungsverfahren zusätzlich Wasser entzogen werden, um die Alkalichlorid-Konzentration zu erhöhen. Bevorzugt ist daher ein Verfahren, dadurch gekennzeichnet, dass die Alkalichlorid-haltige Lösung aus d) vor der Elektrolyse e) mittels Membrandestillationsverfahren oder Umkehrosmose aufkonzentriert wird. Gegenüber Verfahren, bei denen bei der Diarylcarbonatherstellung nur ein Alkalichlorid-haltiges Abwasser mit einem Alkalichlorid-Gehalt von bis zu 17 Gew.-% bezogen auf das Gesamtgewicht der Alkalichlorid-haltigen Lösung aufweist, muss beim erfindungsgemäßen Verfahren jedoch deutlich weniger Wasser durch das Aufkonzentrierungsverfahren entzogen werden, um die gleiche Alkalichlorid-Konzentration zu erreichen.

Hierbei kann beispielsweise die Umkehrosmose oder besonders bevorzugt die Membrandestillation oder Membrankontaktoren eingesetzt werden (siehe MELIN; RAUTENBACH, Membranverfahren; SPRINGER, BERLIN, 2003). Durch Kombination von erfindungsgemäßem Betrieb der Elektrolysezellen und Aufkonzentrierungsverfahren können theoretisch bis zu 100 % des Natriumchlorids aus dem Abwasser wieder gewonnen werden.

In einer weiteren bevorzugten Ausführungsform des erfindungsgemäßen Verfahrens wird das Alkalichlorid-haltige Abwasser der Diarylcarbonatherstellung durch festes Alkalichlorid aufkonzentriert und der Alkalichlorid-Elektrolyse zugeführt. Mit dieser bevorzugten Ausführungform des erfindungsgemäßen Verfahrens könnten mehr als 50% des Alkalichlorids aus dem Abwasser der Diarylcarbonatherstellung recycliert und damit wieder verwendet werden. Dies setzt jedoch voraus, dass das Chlor und die Alkalilauge nicht ausschließlich für die Diarylcarbonat-Produktion eingesetzt werden. Gegenüber Verfahren, bei denen bei der Diarylcarbonatherstellung nur ein Alkalichlorid-haltiges Abwasser mit einem Alkalichlorid-Gehalt von bis zu 17 Gew.-% bezogen auf das Gesamtgewicht der Alkalichlorid-haltigen Lösung aufweist, ist beim erfindungsgemäßen Verfahren jedoch deutlich weniger Zusatz von festem Alkalichlorid zur Aufkonzentrierung erforderlich.

Das erfindungsgemäße Verfahren kann auch mit einer Alkalichlorid-Elektrolyse durchgeführt werden, bei der kein Wasserstoff an der Kathode erzeugt wird, sondern die Kathode durch eine Gasdiffusionselektrode ersetzt wird, an der Sauerstoff zu Hydroxid-Ionen reduziert wird. Wenn z.B. in einem Verbundstandort kein Wasserstoff für chemische Reaktionen benötigt wird, kann auf das Zwangsanfallprodukt Wasserstoff verzichtet werden. Vorteil ist eine Energieeinsparung bei der Elektrolyse, die durch die niedrigere Elektrolysespannung bei Einsatz einer Gasdiffusionselektrode zurückzuführen ist.

Die Alkalichlorid-Elektrolyse sollte üblicherweise so betrieben werden, dass die Alkalichlorid-Konzentration der aus der Zelle gelangenden Alkalichlorid-Lösung zwischen 100 bis 280 g/l Natriumchlorid und/oder dass die Konzentration der Alkalilauge, die aus der Zelle gelangt 13 bis 33 Gew.-% beträgt. Besonders bevorzugt sind Konzentrationen, die den Betrieb der Zelle bei niedrigeren Spannungen ermöglichen. Hierzu sollte die Konzentration der aus der Zelle gelangenden Alkalichlorid-Lösung vorzugsweise zwischen 110 bis 220 g/l Alkalichlorid und/oder die Konzentration der Alkalilauge, die aus der Zelle gelangt 20 bis 30 Gew.-% betragen. In einer bevorzugten Ausführungsform des erfindungsgemäßen Verfahrens wird die Alkalichlorid-Elektrolyse so betrieben, dass die Alkalichlorid-Lösung, die aus der Zelle gelangt, eine NaCl-Konzentration von weniger als 200 g/l aufweist. Parallel hierzu kann die aus der Zelle ablaufende Laugenkonzentration weniger als 30 Gew.-% betragen.

Der Wassertransport über die Membran hängt nicht nur von den Betriebsparametern ab, sondern auch von dem eingesetzten Membran-Typ. Gemäß dem erfindungsgemäßen Verfahren werden bevorzugt solche Ionenaustauschermembranen eingesetzt, die unter den erfindungsgemäßen Bedingungen der Natriumchlorid- und Laugekonzentration einen Wassertransport durch die Membran von mehr als 4,5 mol Wasser je mol Natrium ermöglichen.

Die Stromdichte wird dabei auf die Membranfläche berechnet und beträgt insbesondere 2 bis 6 kA/m². Besonders bevorzugt werden Anoden mit größerer Oberfläche eingesetzt. Unter Anoden mit größerer Oberfläche sind solche zu verstehen, bei denen die physikalische Oberfläche deutlich höher ist als die geometrische, d.h. projizierte Oberfläche. Anoden mit größerer Oberfläche sind z.B. schaum- oder filzartig aufgebaute Elektroden. Hierdurch wird anodisch eine sehr große Elektrodenoberfläche angeboten und die lokale Stromdichte stark erniedrigt. Die Oberfläche der Anode ist bevorzugt so zu wählen, dass die lokale Stromdichte bezogen auf die physikalische Oberfläche der Elektrode weniger als 3 kA/m² beträgt. Je größer die Oberfläche und je niedriger die lokale Stromdichte, desto gering kann die Alkalichlorid-Konzentration in der Sole gewählt werden und desto höher ist der Anteil an Alkalichlorid aus dem Abwasser, das recycelt werden kann.

Der pH-Wert des Alkalichlorid-haltigen Abwassers sollte vor der Elektrolyse e) bevorzugt kleiner als 7 sein, besonders bevorzugt einen Wert von 0,5 bis 6 haben. Die pH-Wert Einstellung kann durch Zugabe von Protonensäuren, bevorzugt Salzsäure oder gasförmigen Chlorwasserstoff erfolgen.

Die eingesetzten Ionenaustauschermembranen bei der Elektrolyse sollten bevorzugt einen Wassertransport je mol Natrium von mehr als 4,0 mol H₂O/mol Natrium aufweisen, besonders bevorzugt 5,5 bis 6,5 mol H₂O/mol Natrium.

Das Verfahren wird bevorzugt so betrieben, dass die Elektrolyse e) bei einer Temperatur von 70 bis 100°C, vorzugsweise bei 80 bis 95°C betrieben wird.

Die Elektrolyse wird bei einem Absolutdruck von 1 bis 1,4 bar, bevorzugt bei einem von 1,1 bis 1,2 bar betrieben. Die Druckverhältnisse zwischen Anoden- und Kathodenraum werden insbesondere so gewählt, dass der Druck im Kathodenraum höher ist als der Druck im Anodenraum. Der Differenzdruck zwischen Kathoden- und Anodenraum sollte in einem besonders bevorzugten Verfahren 20 bis 150 mbar, vorzugsweise 30 bis 100 mbar betragen.

Bei niedrigeren Alkalichlorid-Konzentrationen können auch spezielle Anodencoatings eingesetzt werden. Insbesondere kann das Coating der Anode neben Rutheniumoxid auch weitere Edelmetallkomponenten der 7. und 8. Nebengruppe des Periodensystems der Elemente enthalten. Beispielsweise kann das Anodencoating mit Palladiumverbindungen dotiert werden. Ebenso sind Coatings auf Basis von Diamanten einsetzbar.

Die üblicherweise gemäß bekannten Verfahren aus der Diphenylcarbonat-Herstellung (DPC-Herstellung) erhaltene Natriumchlorid-haltige Lösung (Natriumchlorid-haltiges Abwasser) weist einen Natriumchlorid-Gehalt von bis zu 17 Gew.-% auf, soweit es sich um das Reaktionsabwasser handelt. Wird das Reaktionsabwasser zusätzlich mit dem Waschwasser aus der Aufarbeitung gemäß Schritt c) vereinigt so beträgt die NaCl-Konzentration beispielsweise nur noch ca. 13 Gew.-%. Sofern die Elektrolyse das Chlor und die Natronlauge ausschließlich für die DPC-Produktion liefert, kann das Natriumchlorid-haltige Abwasser nur zu einem geringen Teil in der Elektrolyse eingesetzt werden. So können bei den üblichen Ionenaustauschermembranen und den Standardbetriebsparametem der Natriumchlorid-Elektrolyse nur max. 26 % des Natriumchlorids eines 17 Gew.%igen Natriumchlorid-haltigen DPC-Abwassers eingesetzt werden. Die Standardbetriebsparameter der NaCl-Elektrolyse sind eine Solekonzentration im Ablauf von 200 bis 240g/l und eine NaOH-Konzentration von 31 bis 32 Gew.%. Ein vollständiges Recycling des anfallenden Natriumchlorids ist daher bisher nicht möglich. Eine Aufkonzentrierung durch thermische Verdampfung des Wassers ist zudem derzeit nicht wirtschaftlich, da das Natriumchlorid als sehr preiswertes Produkt zur Verfügung steht.

Mit dem erfindungsgemäßen Verfahren können nun deutlich mehr als 26 % des Natriumchlorid aus anfallenden Abwässern, bei einem NaCl-Gehalt von 18 bis 25 Gew.-% recycelt werden, sofern die Natriumchlorid-Elektrolyse ausschließlich das Chlor und die Natronlauge für die DPC-Produktion bereitstellt. Üblicherweise werden Natriumchlorid-Elektrolysen an chemischen Verbundstandorten mit mehreren Chlorverbrauchern betrieben, so dass nicht von allen Verbrauchern eine Natriumchlorid-haltige Lösung zum Recycling bereit steht. Der Anteil an wieder verwertbarem Natriumchlorid aus dem Abwasser steigt zudem weiter, wenn die Natriumchlorid-Elektrolyse die Natronlauge und das Chlor nicht ausschließlich für die Diarylcarbonat-Produktion bereitstellen muss.

Gegenüber dem Stand der Technik (WO 03/70639 A1), in dem max. 26 % des im Abwasser der DPC-Produktion vorhandenen Natriumchlorids bei der NaCl-Elektrolyse eingesetzt werden kann, können durch das erfindungsgemäße Verfahren mehr als 26 % des Natriumchlorids aus dem Abwasser zurück gewonnen werden.

Die nachfolgenden Beispiele sollen die vorliegende Erfindung illustrieren ohne sie jedoch einzuschränken.

### Beispiele

Die Beispiele sollen das erfindungsgemäße Verfahren anhand des bei der Herstellung von Diphenylcarbonat (DPC-Herstellung) anfallenden Natriumchlorid-haltigen Abwassers darstellen.

### Beispiel 1

### Zusatz von angereichertem Natriumchlorid-haltigem Reaktionsabwasser in die Natriumchlorid-Elektrolyse - Zugabe einer 22,5 Gew.-%igen Natriumchlorid-Lösung aus der DPC-Herstellung zur Natriumchlorid-Elektrolyse

### a) Isolierung des Produktionsabwassers aus der DPC-Herstellung

In einen senkrecht stehenden, gekühlten Rohrreaktor wurde kontinuierlich ein Gemisch aus 145,2 kg/h 14,5%iger Natronlauge, hergestellt durch Verdünnung von 65,8 kg/h einer 32%-igen Natronlauge mit 79,4 kg/h vollentsalztem Wasser (VE-Wasser), und 48,3 kg/h Phenol mit einer Lösung von 86,2 kg/h Methylenchlorid und 27,5 kg/h Phosgen (8 Mol% Überschuss bzgl. auf Phenol) zusammengebracht. Dieses Reaktionsgemisch wurde auf eine Temperatur von 33°C gekühlt und nach einer mittleren Verweilzeit von 15 Sekunden ein pH-Wert von 11,5 gemessen. Zu diesem Reaktionsgemisch wurden nun in der zweiten Stufe des Verfahrens 5,4 kg/h 50%iger NaOH zudosiert, so dass der pH-Wert der zweiten Reaktionsstufe nach einer weiteren Verweilzeit von 5 Minuten 8,5 betrug. In der kontinuierlich betriebenen Reaktion wurden auftretende Dosierschwankungen durch jeweilige Anpassungen der NaOH-Dosierungen aufgefangen. In der zweiten Stufe des Verfahrens wurde das Reaktionsgemisch durch das Durchleiten eines mit Verengungen versehen Rohres ständig gemischt. Die Reaktionstemperatur wurde nach erneuter Zugabe der NaOH durch Kühlen auf 30°C eingestellt. Nach Abtrennung der organischen von der wässrigen Phase (Reaktionsabwasser) wurde die DPC-Lösung mit 0,6 %-iger Salzsäure und Wasser gewaschen. Nach Entfernung des Lösungsmittels wurde 99,9 %-iges Diphenylcarbonat erhalten. Das Reaktionsabwasser wurde mit den Waschphasen vereinigt und durch Strippen mit Wasserdampf von Lösungsmittelresten und Katalysator befreit. Nach Neutralisierung mit Salzsäure und Behandlung mit Aktivkohle enthielt das Produktionsabwasser 13,2 Gew.-% NaCl und weniger als 2 ppm Phenol.

Es konnte ohne weitere Reinigung in das Herstellungsprozess zurückgeführt werden.

### b) Natriumchlorid-Anreicherung im Abwasser durch Rückführung des Abwassers aus a) in die DPC-Herstellung

Die DPC-Herstellung wurde wie in Beispiel 1 beschrieben durchgeführt, jedoch wurde statt 79,4 kg/h VE-Wasser kontinuierlich eine Mischung aus 37,9 kg/h VE-Wasser und 47,7 kg/h des nach Beispiel 1a) erhaltenen Produktionsabwassers eingesetzt.

Nach Abtrennung der organischen von der wässrigen Phase (Reaktionsabwasser) wurde die organische Phase mit 0,6 %-iger Salzsäure und Wasser gewaschen. Nach Entfernung des Lösungsmittels wurde 99,9 %-iges Diphenylcarbonat erhalten. Das Reaktionsabwasser wurde ohne vorherige Vereinigung mit den Waschphasen durch Strippen mit Wasserdampf von Lösungsmittelresten und Katalysator befreit. Nach Neutralisierung mit Salzsäure und Behandlung mit Aktivkohle enthielt das Reaktionsabwasser 22,5 Gew.-% NaCl und weniger als 2 ppm Phenol.

### c) Elektrochemische Oxidation des Reaktionsabwassers aus b)

Die Elektrolyse wurde beispielhaft in einer Laborelektrolysezelle mit einer Anodenfläche von 0,01 m² durchgeführt. Die Stromdichte betrug 4 kA/m², Temperatur Auslauf Kathodenseite 88°C, Temperatur Auslauf Anodenseite 89°C. Eingesetzt wurde eine Elektrolysezelle mit Standard Anoden- und Kathodencoating der Fa. DENORA, Deutschland. Eingesetzt wurde eine Ionenaustauschermembran der Fa. DuPont Nafion 982 WX. Die Elektrolysespannung betrug 3,02 V. Durch die Anodenkammer wurde eine Natriumchlorid-haltige Lösung mit einem Massenstrom von 0,96 kg/h umgepumpt. Die Konzentration der der Anodenkammer zugeführten Lösung betrug 25 Gew.-% NaCl. Aus der Anodenkammer konnte eine 20 Gew.%ige NaCl-Lösung entnommen werden. Der aus der Anodenkammer entnommenen NaCl-Lösung wurden 0,14 kg/h an 22,5 Gew.-%igen Reaktionsabwasser aus der Diphenylcarbonatherstellung unter b) und 0,0543 kg/h festes Natriumchlorid zugegeben. Die Lösung wurde anschließend wieder in die Anodenkammer eingespeist. Der Wassertransport über die Membran betrug 3,8 mol Wasser je mol Natrium.

Auf der Kathodenseite wurde eine Natronlauge mit einem Massenstrom von 0,849 kg/h umgepumpt. Die Konzentration der in die Kathodenseite eingespeisten Natronlauge betrug 30 Gew.-% NaOH, die aus der Kathodenseite entnommene Natronlauge hatte eine Konzentration von 32,1 % NaOH. 0,186 kg/h der 32,1%igen Lauge wurden dem Volumenstrom entnommen, der Rest wurde mit 0,057 kg/h Wasser aufgestockt und wieder in das Kathodenelement zurückgeführt.

35,7 % des umgesetzten Natriumchlorids stammten aus dem Reaktionsabwasser der DPC-Herstellung.

### Beispiel 2

### Zusatz von angereichertem Natriumchlorid-haltigem Produktionsabwasser in die Natriumchlorid-Elektrolyse - Zugabe einer 18,0 Gew.-%igen Natriumchlorid-Lösung aus der DPC-Herstellung zur Natriumchlorid-Elektrolyse

### a) Kochsalzanreicherung von Produktionsabwasser durch Abwasserrückführung in das Herstellungsprozess

Es wurde wie in Beispiel 1b) beschrieben verfahren, jedoch wurde das Reaktionsabwasser mit den Waschphasen vereinigt und anschließend durch Strippen mit Wasserdampf von Lösungsmittelresten und Katalysator befreit. Nach Neutralisierung mit Salzsäure und Behandlung mit Aktivkohle enthielt das Produktionsabwasser 18,0 Gew.-% NaCl und weniger als 2 ppm organische Verunreinigungen.

Es konnte ohne weitere Reinigung der elektrochemische Oxidation zu Chlor zugeführt werden.

### b) Elektrochemische Oxidation des angereicherten Produktionsabwassers aus a)

Die Elektrolyse wurde beispielhaft in einer Laborelektrolysezelle mit einer Anodenfläche von 0,01 m² durchgeführt. Die Stromdichte betrug 4 kA/m², Temperatur Auslauf Kathodenseite 88°C, Temperatur Auslauf Anodenseite 89°C. Eingesetzt wurde eine Elektrolysezelle mit Standard Anoden- und Kathodencoating der Fa. DENORA, Deutschland. Eingesetzt wurde eine Ionenaustauschermembran der Fa. DuPont Nafion 982 WX. Die Elektrolysespannung betrug 3,02 V. Durch die Anodenkammer wurde eine Natriumchlorid-haltige Lösung mit einem Massenstrom von 0,96 kg/h umgepumpt. Die Konzentration der der Anodenkammer zugeführten Lösung betrug 25 Gew.-% NaCl. Aus der Anodenkammer konnte eine 20 Gew.%ige NaCl-Lösung entnommen werden. Der aus der Anodenkammer entnommenen NaCl-Lösung wurden 0,133 kg/h an 18 Gew.-%igem Produktionsabwasser aus der Diphenylcarbonatherstellung unter a) und 0,062 kg/h festes Natriumchlorid zugegeben. Die Lösung wurde anschließend wieder in die Anodenkammer eingespeist. Der Wassertransport über die Membran betrug 3,8 mol Wasser je mol Natrium.

Auf der Kathodenseite wurde eine Natronlauge mit einem Massenstrom von 0,849 kg/h umgepumpt. Die Konzentration der in die Kathodenseite eingespeisten Natronlauge betrug 30 Gew.-% NaOH, die aus der Kathodenseite entnommene Natronlauge hatte eine Konzentration von 32,2 % NaOH. 0,185 kg/h der 32,2%igen Lauge wurden dem Volumenstrom entnommen, der Rest wurde mit 0,057 kg/h Wasser aufgestockt und wieder in das Kathodenelement zurückgeführt.

27,0 % des umgesetzten Natriumchlorids stammten aus dem DPC-Produktionsabwasser.

### Beispiel 3

### Zusatz von angereichertem Natriumchlorid-haltigem Reaktionsabwasser in die Natriumchlorid-Elektrolyse - Zugabe einer 22,3 Gew.-%igen Natriumchlorid-Lösung aus der DPC-Herstellung zur Natriumchlorid-Elektrolyse

### a) Kochsalzanreicherung von Reaktionsabwasser durch Erhöhung_ der Natriumphenolatkonzentration bei der DPC-Herstellung

Es wurde wie in Beispiel 1a) beschrieben verfahren, jedoch wurde statt 79,4 kg/h nur 54,6 kg/h VE-Wasser eingesetzt. Nach Abtrennung der organischen von der wässrigen Phase (Reaktionsabwasser) wurde die organische Phase mit 0,6 %-iger Salzsäure und Wasser gewaschen. Nach Entfernung des Lösungsmittels wurde 99,9 %-iges Diphenylcarbonat erhalten. Das Reaktionsabwasser wurde ohne vorherige Vereinigung mit den Waschphasen durch Strippen mit Wasserdampf von Lösungsmittelresten und Katalysator befreit. Nach Neutralisierung mit Salzsäure und Behandlung mit Aktivkohle enthielt das Reaktionsabwasser 22,3 Gew.-% NaCl und weniger als 2 ppm Phenol.

### b) Elektrochemische Oxidation des angereicherten Reaktionsabwassers aus a)

Die Elektrolyse wurde beispielhaft in einer Laborelektrolysezelle mit einer Anodenfläche von 0,01 m² durchgeführt. Die Stromdichte betrug 4 kA/m², Temperatur Auslauf Kathodenseite 88°C, Temperatur Auslauf Anodenseite 89°C. Eingesetzt wurde eine Elektrolysezelle mit Standard Anoden- und Kathodencoating der Fa. DENORA, Deutschland. Eingesetzt wurde eine Ionenaustauschermembran der Fa. DuPont Nafion 982 WX. Die Elektrolysespannung betrug 3,02 V. Durch die Anodenkammer wurde eine Natriumchlorid-haltige Lösung mit einem Massenstrom von 0,96 kg/h umgepumpt. Die Konzentration der der Anodenkammer zugeführten Lösung betrug 25 Gew.-% NaCl. Aus der Anodenkammer konnte eine 20 Gew.-%ige NaCl-Lösung entnommen werden. Der aus der Anodenkammer entnommenen NaCl-Lösung wurden 0,14 kg/h an 22,3 Gew.-%igem Reaktionsabwasser aus der Diphenylcarbonatherstellung unter a) und 0,0546 kg/h festes Natriumchlorid zugegeben. Die Lösung wurde anschließend wieder in die Anodenkammer eingespeist. Der Wassertransport über die Membran betrug 3,8 mol Wasser je mol Natrium.

Auf der Kathodenseite wurde eine Natronlauge mit einem Massenstrom von 0,849 kg/h umgepumpt. Die Konzentration der in die Kathodenseite eingespeisten Natronlauge betrug 30 Gew.-% NaOH, die aus der Kathodenseite entnommene Natronlauge hatte eine Konzentration von 32,2 % NaOH. 0,186 kg/h der 32,2%igen Lauge wurden dem Volumenstrom entnommen, der Rest wurde mit 0,057 kg/h Wasser aufgestockt und wieder in das Kathodenelement zurückgeführt.

35,3% des umgesetzten Natriumchlorids stammten aus dem DPC-Reaktionsabwasser.

### Beispiel 4 (Vergleich)

### Zusatz von Natriumchlorid-haltigem Reaktionsabwasser in die Natriumchlorid-Elektrolyse - Zugabe einer 17 Gew.-%igen Natriumchlorid-Lösung aus der DPC-Herstellung

### a) Isolierung des Reaktionsabwassers aus der DPC-Herstellung

In einen senkrecht stehenden, gekühlten Rohrreaktor wurde kontinuierlich ein Gemisch aus 145,2 kg/h 14,5%iger Natronlauge, hergestellt durch Verdünnung von 65,8 kg/h einer 32%-igen Natronlauge mit 79,4 kg/h vollentsalztem Wasser (VE-Wasser), und 48,3 kg/h Phenol mit einer Lösung von 86,2 kg/h Methylenchlorid und 27,5 kg/h Phosgen (8 Mol% Überschuss bzgl. auf Phenol) zusammengebracht. Dieses Reaktionsgemisch wurde auf eine Temperatur von 33°C gekühlt und nach einer mittleren Verweilzeit von 15 Sekunden ein pH-Wert von 11,5 gemessen. Zu diesem Reaktionsgemisch wurden nun in der zweiten Stufe des Verfahrens 5,4 kg/h 50%iger NaOH zudosiert, so dass der pH-Wert der zweiten Reaktionsstufe nach einer weiteren Verweilzeit von 5 Minuten 8,5 betrug. In der kontinuierlich betriebenen Reaktion wurden auftretende Dosierschwankungen durch jeweilige Anpassungen der NaOH-Dosierungen aufgefangen. In der zweiten Stufe des Verfahrens wurde das Reaktionsgemisch durch das Durchleiten eines mit Verengungen versehen Rohres ständig gemischt. Die Reaktionstemperatur wurde nach erneuter Zugabe der NaOH durch Kühlen auf 30°C eingestellt. Nach Abtrennung der organischen von der wässrigen Phase (Reaktionsabwasser) wurde die DPC-Lösung mit 0,6 %-iger Salzsäure und Wasser gewaschen. Nach Entfernung des Lösungsmittels wurde 99,9 %-iges Diphenylcarbonat erhalten. Das Reaktionsabwasser wurde nicht mit den Waschphasen vereinigt und wurde durch Strippen mit Wasserdampf von Lösungsmittelresten und Katalysator befreit. Nach Neutralisierung mit Salzsäure und Behandlung mit Aktivkohle enthielt das Reaktionsabwasser 17 Gew.-% NaCl und weniger als 2 ppm Phenol.

Es konnte ohne weitere Reinigung der Natriumchlorid-Elektrolysezelle zugeführt werden.

### b) Elektrochemische Oxidation des Reaktionsabwassers aus a)

Die Elektrolyse wurde beispielhaft in einer Laborelektrolysezelle mit einer Anodenfläche von 0,01 m² durchgeführt. Die Stromdichte betrug 4 kA/m², Temperatur Auslauf Kathodenseite 88°C, Temperatur Auslauf Anodenseite 89°C. Eingesetzt wurde eine Elektrolysezelle mit Standard Anoden- und Kathodencoating der Fa. DENORA, Deutschland. Eingesetzt wurde eine Ionenaustauschermembran der Fa. DuPont Nafion 982 WX. Die Elektrolysespannung betrug 3,02 V. Durch die Anodenkammer wurde eine Natriumchlorid-haltige Lösung mit einem Massenstrom von 0,98 kg/h umgepumpt. Die Konzentration der der Anodenkammer zugeführten Lösung betrug 25 Gew.-% NaCl. Aus der Anodenkammer konnte eine 20 Gew.%ige NaCl-Lösung entnommen werden. Der aus der Anodenkammer entnommenen NaCl-Lösung wurden 0,121 kg/h an 17 Gew.-%igen Reaktionsabwasser aus der Diphenylcarbonatherstellung aus Beispiel 4a) und 0,0653 kg/h festes Natriumchlorid zugegeben. Die Lösung wurde anschließend wieder in die Anodenkammer eingespeist. Der Wassertransport über die Membran betrug 3,5 mol Wasser je mol Natrium.

Auf der Kathodenseite wurde eine Natronlauge mit einem Massenstrom von 1,107 kg/h umgepumpt. Die Konzentration der in die Kathodenseite eingespeisten Natronlauge betrug 30 Gew.-% NaOH, die aus der Kathodenseite entnommene Natronlauge hatte eine Konzentration von 32 % NaOH. 0,188 kg/h der 31,9%igen Lauge wurden dem Volumenstrom entnommen, der Rest wurde mit 0,0664 kg/h Wasser aufgestockt und wieder in das Kathodenelement zurückgeführt.

Lediglich 23,3 % des umgesetzten Natriumchlorids stammten aus dem DPC-Reaktionsabwasser.

Die vorangehenden erfindungsgemäßen Beispiele 1 bis 3 zeigen im Vergleich zum Vergleichsbeispiel, dass durch die Aufkonzentrierung des Natriumchlorid-haltigen Abwassers sowohl der Anteil des während der nachfolgenden Elektrolyse umgesetzten Natriumchlorids, welches aus der DPC-Herstellung stammt, deutlich gesteigert wird als auch die Menge des zusätzlich zuzugebenden Natriumchlorids im fester Form vor der Elektrolyse deutlich verringert werden kann. Dies resultiert in einer deutlich besseren Verwertung des Natriumchlorids im Abwasser und geringerer Mengen zu entsorgender, salzbelasteter Prozessabwässer.

## Patentansprüche

1. Verfahren zur Herstellung von Diarylcarbonat und Verarbeitung mindestens eines Teils der dabei anfallenden Alkalichlorid-haltigen Lösung in einer nachgeschalteten Alkalichlorid-Elektrolyse, umfassend die folgenden Schritte:
a) Herstellung von Phosgen durch Umsetzung von Chlor mit Kohlenmonoxid, und
b) Umsetzung des gemäß Schritt a) gebildeten Phosgens mit wenigstens einem Monophenol in Gegenwart einer Base und gegebenenfalls basischem Katalysator zu einem Diarylcarbonat und einer Alkalichlorid-haltigen Lösung, und
c) Abtrennung und Aufarbeitung des in Schritt b) gebildeten Diarylcarbonats, und
d) Abtrennung der gemäß Schritt c) verbliebenen Alkalichlorid-haltigen Lösung von Lösungsmittelresten und gegebenenfalls Katalysatorresten, insbesondere durch Strippen der Lösung mit Wasserdampf, und Behandlung mit Adsorbentien, insbesondere mit Aktivkohle, wobei vor der Behandlung mit Adsorbentien die Alkalichlorid-haltige Lösung auf einen pH-Wert kleiner oder gleich 8 eingestellt wird, und
e) Elektrochemische Oxidation wenigstens eines Teils der Alkalichlorid-haltigen Lösung aus d) unter Bildung von Chlor, Alkalilauge und ggf. Wasserstoff, und
f) Rückführung wenigstens eines Teil des gemäß Schritt e) hergestellten Chlors in die Herstellung von Phosgen gemäß Schritt a) und/oder
g) Rückführung wenigstens ein Teil des gemäß Schritt e) hergestellten Alkalilauge in die Herstellung von Diarylcarbonat gemäß Schritt b),
**dadurch gekennzeichnet, dass** die in Schritt b) hergestellte Alkalichlorid-haltige Lösung einen Alkalichlorid-Gehalt von 18 bis 25 Gew.-% bezogen auf das Gesamtgewicht der Alkalichlorid-haltigen Lösung hat und/oder wenigstens ein Teil der in Schritt d) anfallenden Alkalichlorid-haltigen Lösung in Schritt b) zurückgeführt wird.

2. Verfahren gemäß Anspruch 1, **dadurch gekennzeichnet, dass** in Schritt b) als Base eine Alkali-haltige Base, bevorzugt eine Natrium-haltige Base, eingesetzt wird und diese Alkalihaltige Base und Monophenol in solchen Mengen eingesetzt werden, dass der Natriumphenolatgehalt der resultierenden Lösung aus Alkali-haltiger Base und Monophenol 31 bis 40 Gew.-% Natriumphenolat, bezogen auf das Gesamtgewicht der Lösung, beträgt.

3. Verfahren gemäß Anspruch 1 und 2, **dadurch gekennzeichnet, dass** bis zu 80 Gew.-%, bevorzugt bis zu 50 Gew.-% der in Schritt d) anfallenden Alkalichlorid-haltigen Lösung in Schritt b) zurückgeführt werden.

4. Verfahren gemäß wenigstens einem der Ansprüche 1 bis 3, **dadurch gekennzeichnet, dass** die elektrochemische Oxidation wenigstens eines Teiles der Alkalichlorid-haltigen Lösung aus d) zu Chlor und Natronlauge unter Einsatz von Gasdiffusionselektroden als Kathode erfolgt.

5. Verfahren gemäß einem der Ansprüche 1 bis 4, **dadurch gekennzeichnet, dass** wenigstens ein Teil der gereinigten Alkalichlorid-haltigen Lösung aus d) in den Solekreislauf einer Membranelektrolyse zur Herstellung von Chlor, Natronlauge und gegebenenfalls Wasserstoff gegeben wird.

6. Verfahren gemäß einem der Ansprüche 1 bis 5, **dadurch gekennzeichnet, dass** der Alkalichlorid-haltigen Lösung bei der Elektrolyse e) zusätzliches Alkalichlorid zur Erhöhung der Alkalichlorid-Konzentration zugegeben wird.

7. Verfahren gemäß einem der Ansprüche 1 bis 6, **dadurch gekennzeichnet, dass** die Alkalichlorid-haltige Lösung vor der Behandlung mit Adsorbentien in Schritt d) auf einen pH-Wert von kleiner als 7 eingestellt wird, insbesondere durch Einsatz von Salzsäure oder Chlorwasserstoff.

8. Verfahren gemäß einem der Ansprüche 1 bis 7, **dadurch gekennzeichnet, dass** bei der Elektrolyse e) Ionenaustauschermembranen eingesetzt werden, deren Wassertransport je mol Natrium größer als 4 mol H₂O/mol Natrium, bevorzugt von 5,5 bis 6,5 mol H₂O/mol Natrium ist.

9. Verfahren gemäß einem der Ansprüche 1 bis 8, **dadurch gekennzeichnet, dass** als Monophenol in Stufe b) Phenol, C₁-C₉-Alkylphenole, insbesondere Kresole, p-tert.-Butylphenol, p-Cumylphenol, p-n-Octylphenol, p-iso-Octylphenol, p-n-Nonylphenol und p-isoNonylphenol, Halogenphenole insbesondere p-Chlorphenol, 2,4-Dichlorphenol, p-Bromphenol und 2,4,6-Tribromphenol, besonders bevorzugt Phenol eingesetzt wird.

10. Verfahren gemäß einem der Ansprüche 1 bis 9, **dadurch gekennzeichnet, dass** in der Elektrolyse e) in den Zellen Anoden mit größerer Oberfläche als die Oberfläche der Membranen eingesetzt werden.

## Claims

1. Process for production of diaryl carbonate and processing of at least a part of the resultant alkali-metal-chloride-containing solution in a downstream alkali metal chloride electrolysis, comprising the following steps:
a) producing phosgene by reacting chlorine with carbon monoxide, and
b) reacting the phosgene formed according to step a) with at least one monophenol in the presence of a base and optionally basic catalyst to give a diaryl carbonate and an alkali-metal-chloride-containing solution, and
c) separating off and working up the diaryl carbonate formed in step b), and
d) separating off the alkali-metal-chloride-containing solution remaining according to step c) from solvent residues and optionally catalyst residues, in particular by stripping the solution with steam, and treating it with adsorbents, in particular with activated carbon, wherein, before the treatment with adsorbents, the alkali-metal-chloride-containing solution is adjusted to a pH less than or equal to 8, and
e) electrochemically oxidizing at least a part of the alkali-metal-chloride-containing solution from d) with formation of chlorine, alkali solution and optionally hydrogen, and
f) recycling at least a part of the chlorine produced according to step e) to the production of phosgene according to step a) and/or
g) recycling at least a part of the alkali solution produced according to step e) to the production of diaryl carbonate according to step b),
**characterized in that** the alkali-metal-chloride-containing solution produced in step b) has an alkali metal chloride content of 18 to 25% by weight based on the total weight of the alkali-metal-chloride-containing solution and/or at least a part of the alkali-metal-chloride-containing solution occurring in step d) is recycled to step b).

2. Process according to Claim 1, **characterized in that**, in step b), the base is an alkali-metal-containing base, preferably a sodium-containing base, and this alkali-metal-containing base and monophenol are used in amounts such that the sodium phenolate content of the resultant solution of alkali-metal-containing base and monophenol is 31 to 40% by weight sodium phenolate, based on the total weight of the solution.

3. Process according to Claim 1 and 2, **characterized in that** up to 80% by weight, preferably up to 50% by weight, of the alkali-metal-chloride-containing solution occurring in step d) is recycled to step b).

4. Process according to at least one of Claims 1 to 3, **characterized in that** the electrochemical oxidation of at least a part of the alkali-metal-chloride-containing solution from d) to give chlorine and sodium hydroxide solution proceeds with the use of gas diffusion electrodes as cathode.

5. Process according to one of Claims 1 to 4, **characterized in that** at least a part of the purified alkali-metal-chloride-containing solution from d) is added to the brine circuit of a membrane electrolysis for producing chlorine, sodium hydroxide solution and optionally hydrogen.

6. Process according to one of Claims 1 to 5, **characterized in that** additional alkali metal chloride is added to the alkali-metal-chloride-containing solution in the electrolysis e) for elevation of the alkali metal chloride concentration.

7. Process according to one of Claims 1 to 6, **characterized in that** the alkali-metal-chloride-containing solution, before the treatment with adsorbents in step d), is adjusted to a pH of less than 7, in particular by using hydrochloric acid or hydrogen chloride.

8. Process according to one of Claims 1 to 7, **characterized in that**, in the electrolysis e), ion-exchange membranes are used, the water transport of which per mole of sodium is greater than 4 mol of H₂O/mole of sodium, preferably 5.5 to 6.5 mol of H₂O/mole of sodium.

9. Process according to one of Claims 1 to 8, **characterized in that** the monophenol in stage b) is phenol, C₁-C₉-alkylphenols, in particular cresols, p-tert-butylphenol, p-cumylphenol, p-n-octylphenol, p-isooctylphenol, p-n-nonylphenol and p-isononylphenol, halophenols, in particular p-chlorophenol, 2,4-dichlorophenol, p-bromophenol and 2,4,6-tribromophenol, particularly preferably phenol.

10. Process according to one of Claims 1 to 9, **characterized in that**, in the electrolysis e), in the cells, use is made of anodes having a higher surface area than the surface area of the membranes.

## Revendications

1. Procédé pour la production de diarylcarbonate et transformation d'au moins une partie de la solution contenant du chlorure de métal alcalin obtenue dans ce cas dans une électrolyse de chlorure de métal alcalin disposée en aval, comprenant les étapes suivantes :
a) production de phosgène par réaction de chlore avec du monoxyde de carbone, et
b) transformation du phosgène formé selon l'étape a) avec au moins un monophénol en présence d'une base et le cas échéant d'un catalyseur basique en un diarylcarbonate et une solution contenant du chlorure de métal alcalin, et
c) séparation et traitement de diarylcarbonate formé dans l'étape b), et
d) séparation de la solution contenant du chlorure de métal alcalin résiduelle selon l'étape c) de restes de solvant et le cas échéant de restes de catalyseur, en particulier par rectification de la solution avec de la vapeur d'eau et traitement avec des adsorbants, en particulier du charbon actif, en réglant, avant le traitement avec les adsorbants la solution contenant du chlorure de métal alcalin à un pH inférieur ou égal à 8, et
e) oxydation électrochimique d'au moins une partie de la solution contenant du chlorure de métal alcalin de d) avec formation de chlore, de lessive alcaline et le cas échéant d'hydrogène, et
f) recyclage d'au moins une partie du chlore produit selon l'étape e) dans la production de phosgène selon l'étape a) et/ou
g) recyclage d'au moins une partie de la lessive alcaline produite selon l'étape e) dans la production de diarylcarbonate selon l'étape b),
**caractérisé en ce que** la solution contenant du chlorure de métal alcalin produite dans l'étape b) présente une teneur en chlorure de métal alcalin de 18 à 25% en poids par rapport au poids total de la solution contenant du chlorure de métal alcalin et/ou au moins une partie de la solution contenant du chlorure de métal alcalin obtenue dans l'étape d) est recyclée dans l'étape b).

2. Procédé selon la revendication 1, **caractérisé en ce qu'**on utilise dans l'étape b), comme base, une base contenant un métal alcalin, de préférence une base contenant du sodium, et cette base contenant du métal alcalin et le monophénol sont utilisés en des quantités telles que la teneur en phénolate de sodium de la solution de la base contenant du métal alcalin et du monophénol est de 31 à 40% en poids de phénolate de sodium, par rapport au poids total de la solution.

3. Procédé selon la revendication 1 et 2, **caractérisé en ce que** jusqu'à 80% en poids, de préférence jusqu'à 50% en poids de la solution contenant du chlorure de métal alcalin obtenue dans l'étape d) sont recyclés dans l'étape b).

4. Procédé selon au moins l'une quelconque des revendications 1 à 3, **caractérisé en ce que** l'oxydation électrochimique d'au moins une partie de la solution contenant du chlorure de métal alcalin de d) en chlore et en lessive de soude est réalisée avec utilisation d'électrodes à diffusion de gaz comme cathode.

5. Procédé selon l'une quelconque des revendications 1 à 4, **caractérisé en ce qu'**au moins une partie de la solution purifiée contenant du chlorure de métal alcalin de d) est introduite dans la circulation de base d'une électrolyse membranaire pour la production de chlore, de lessive de soude et le cas échéant d'hydrogène.

6. Procédé selon l'une quelconque des revendications 1 à 5, **caractérisé en ce que** la solution contenant du chlorure de métal alcalin est additionnée de chlorure de métal alcalin supplémentaire lors de l'électrolyse e) pour augmenter la concentration en chlorure de métal alcalin.

7. Procédé selon l'une quelconque des revendications 1 à 6, **caractérisé en ce que** la solution contenant du chlorure de métal alcalin est réglée, avant le traitement avec des adsorbants dans l'étape d), à un pH inférieur à 7, en particulier par utilisation d'acide chlorhydrique ou de chlorure d'hydrogène.

8. Procédé selon l'une quelconque des revendications 1 à 7, **caractérisé en ce qu'**on utilise, lors de l'électrolyse e), des membranes échangeuses d'ions dont le transport d'eau par mole de sodium est supérieur à 4 moles de H₂O/mole de sodium, de préférence de 5,5 à 6,5 moles de H₂O/mole de sodium.

9. Procédé selon l'une quelconque des revendications 1 à 8, **caractérisé en ce qu'**on utilise comme monophénol dans l'étape b) le phénol, les C₁-C₉-alkylphénols, en particulier les crésols, le p-tert-butylphénol, le p-cumylphénol, le p-n-octylphénol, le p-iso-octylphénol, le p-n-nonylphénol et le p-isononylphénol, les halogénophénols, en particulier le p-chlorophénol, le 2,4-dichlorophénol, le p-bromophénol et le 2,4,6-tribromophénol, de manière particulièrement préférée le phénol.

10. Procédé selon l'une quelconque des revendications 1 à 9, **caractérisé en ce qu'**on utilise dans l'électrolyse e), dans les cellules, des anodes avec une surface plus grande que la surface des membranes.
